# EUROPEAN PATENT APPLICATION

(11) **EP 1 656 936 A1**
(43) Date of publication of application: **17.05.2006**
(21) Application number: 04771287.2
(22) Date of filing: 05.08.2004
(51) Int. Cl.: A61K 31/222, A61K 31/336, A61P 1/00, A61P 1/02, A61P 1/04, A61P 3/10, A61P 7/00, A61P 9/10, A61P 11/00, A61P 11/06, A61P 17/00, A61P 19/02, A61P 25/00, A61P 25/16, A61P 25/28, A61P 29/00, A61P 31/00, A61P 31/04, A61P 31/12, A61P 31/16, A61P 31/18

(54) **MACROPHAGE ACTIVATION INHIBITOR**

(30) Priority: 06.08.2003 JP 2003288280
(71) Applicant: Signal Creation Inc., Yokohama-shi, Kanagawa 223-0061 (JP)
(72) Inventor: UMEZAWA, Kazuo, Keio Univ. Faculty of Scie & Tech, Yokohama-shi, Kanagawa 223-0061 (JP); SUZUKI, Eriko, Keio Univ. Faculty of Scie & Tech, Yokohama-shi, Kanagawa 223-0061 (JP)
(74) Representative: Horner, Martin Grenville
(86) International application number: PCT/JP2004/011260
(87) International publication number: WO 2005/013966

(57) **Abstract**

A compound represented by the following general formula (1) : 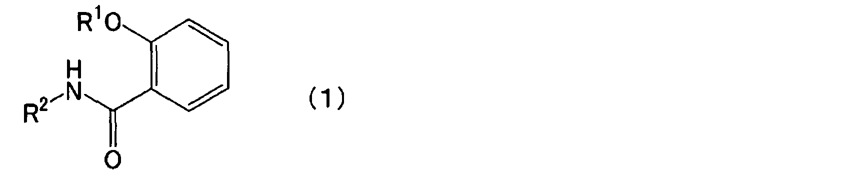 wherein R¹ represents a hydrogen atom or a C2 to C4 alkanoyl group and R² represents a group represented by the following formula (A), (B), (C), (D), (E), (F), or (G): 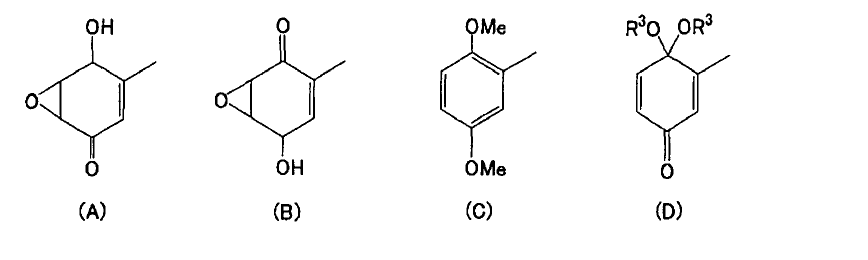 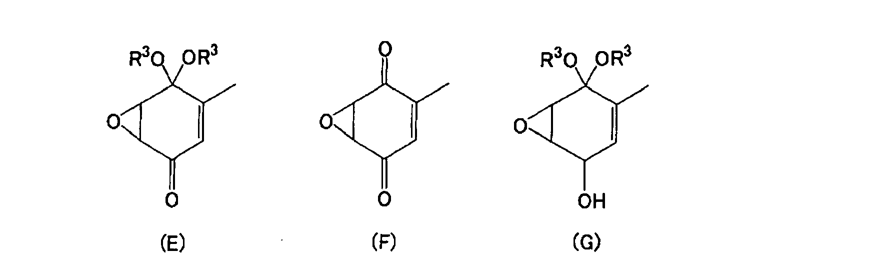 wherein R³ represents a C1 to C4 alkyl group can inhibit macrophage activation. Accordingly, a macrophage activation inhibitor containing a compound represented by the general formula (1) or its pharmacologically acceptable salt as an active ingredient is useful as an agent for preventing, improving, or treating a disease resulting from infection with pathogens such as a bacterium and a virus.

## Description

### TECHNICAL FIELD

The present invention relates to macrophage activation inhibitors, therapeutic agents for infectious diseases, therapeutic agents for type IV allergic diseases, and therapeutic agents for improving severity of atopic disease associated with pathogenic infection.

### BACKGROUND ART

It is known that pathogens, such as bacteria and viruses, invade the living body and cause infectious diseases, such as influenza and Staphylococcus aureus infection. In order to prevent and treat these infectious diseases, various preventive and therapeutic agents have previously been developed (Japanese Laid-Open Application No.8-12574, Japanese Laid-Open Application No.10-251148, and Japanese Laid-Open Application No.10-231247).

It has recently been clarified that these infections are accompanied by activation of macrophage, suggesting that agents capable of suppressing and inhibiting this macrophage activation are useful as therapeutic agents for infection.

### DISCLOSURE OF THE INVENTION

### PROBLEMS TO BE SOLVED BY THE INVENTION

Thus, the object of the present invention is to provide macrophage activation inhibitors useful to prevent, improve, or treat diseases resulting from infection with pathogens, such as bacteria and viruses, and immune diseases, such as allergies.

### MEANS FOR SOLVING THE PROBLEMS

The inventors have assiduously studied in order to provide agents for inhibiting macrophage activation, and as a result, found that a compound (hereinafter referred to as "(±)-DHM2EQ".) represented by the formula (1a) strongly inhibits activation of macrophages stimulated by lipopolysaccharide (LPS). Thus, the present invention has been accomplished.

Thus, the inhibitor for inhibiting macrophage activation according to the present invention contains a compound represented by the following general formula (1) or its pharmacologically acceptable salt as an active ingredient:

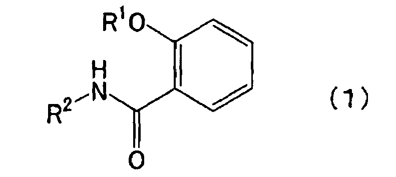

wherein R¹ represents a hydrogen atom or a C2 to C4 alkanoyl group. Examples of the alkanoyl group include an acetyl group, a propionyl group, a butanoyl group, and their isomer groups, of which an acetyl group is particularly preferred.

R² represents a group represented by the following formula (A), (B), (C), (D), (E), (F), or (G) :

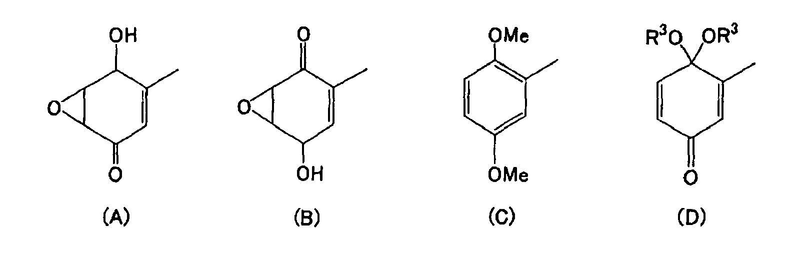

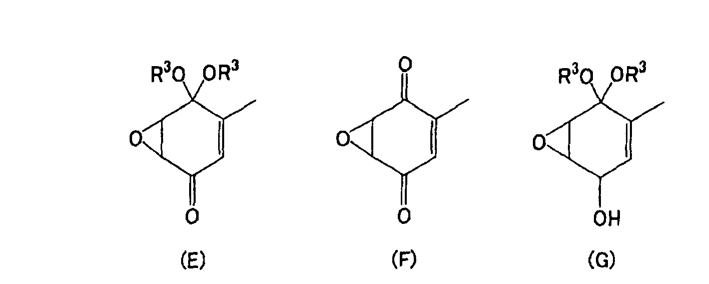

wherein R³ represents a C1 to C4 alkyl group. Examples of the alkyl group include a methyl group, an ethyl group, a propyl group, and butyl group, and their isomer groups, of which a methyl group and an ethyl group are particularly preferred.

Further, the aforementioned compound is preferably (±)-DHM2EQ (the following formula (1a)) or a compound represented by the following formula (1b), (which is hereinafter referred to as "(±)-DHM3EQ"), particularly preferably the following formula (2), and most preferably one that does not contain a levorotatory (+) compound.

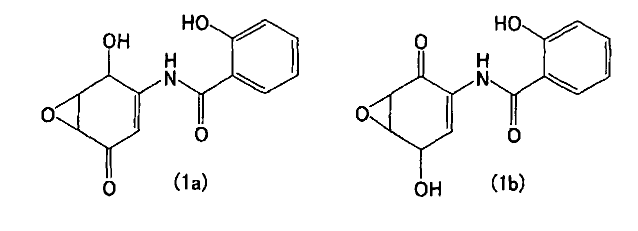

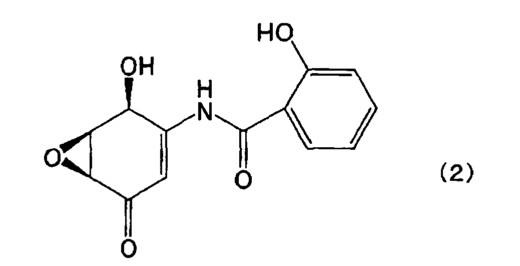

Further, the inhibitor for inhibiting macrophage activation according to the present invention prevents, improves, or treats a disease resulting from macrophage activation.

The aforementioned infectious disease may result from bacterial infection or viral infection.

Further, for the inhibitor for inhibiting macrophage activation according to the present invention, the macrophage activation results from any one factor selected from the group consisting of lipopolysaccharide, IFN-γ, and IL-1β.

It should be noted that macrophages refer to large, monocyte-derived cells having phagocytic ability. Examples of macrophages include free macrophages, blood monocytes, alveolar macrophages, peritoneal macrophages, inflammatory granuloma macrophages, fixed macrophages, Kupffer cells, microglia cells in the central nervous system, histiocytes found in subcutaneous and other connective tissue, dendritic macrophages extending processes into the reticuloendothelial system and lymphatic clefts in the spleen, bone marrow, and the lymph node sinus, vascular adventitial cells, meningeal and perivascular macrophages in the central nervous system, etc.

The therapeutic agent for an infectious disease according to the present invention contains a compound represented by the following general formula (1) or its pharmacologically acceptable salt as an active ingredient:

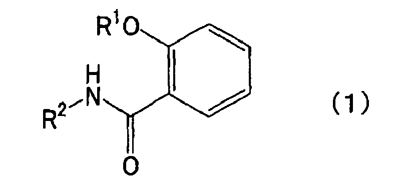

wherein R¹ represents a hydrogen atom or a C2 to C4 alkanoyl group. Examples of the alkanoyl group include an acetyl group, a propionyl group, a butanoyl group, and their isomer groups, of which an acetyl group is particularly preferred.

R² represents a group represented by the following formula (A), (B), (C), (D), (E), (F), or (G) :

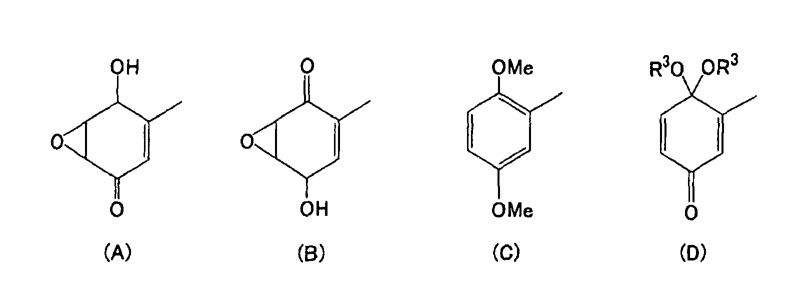

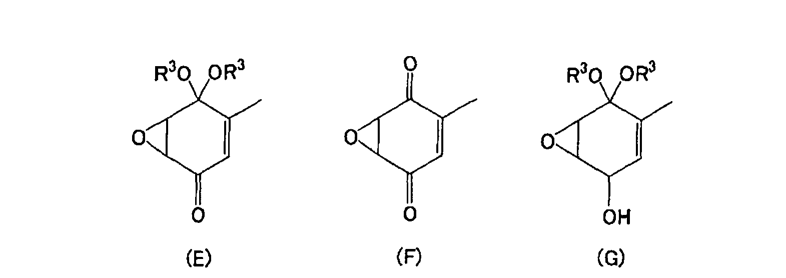

wherein R³ represents a C1 to C4 alkyl group. Examples of the alkyl group include a methyl group, an ethyl group, a propyl group, and butyl group, and their isomer groups, of which a methyl group and an ethyl group are particularly preferred.

Further, the aforementioned compound is preferably (±)-DHM2EQ (the following formula (1a)) or (±)-DHM3EQ (the compound represented by the following formula (1b)), particularly preferably the following formula (2), and most preferably one that substantially does not contain a levorotatory (+) compound.

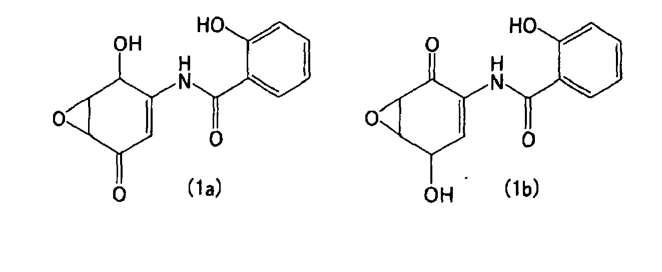

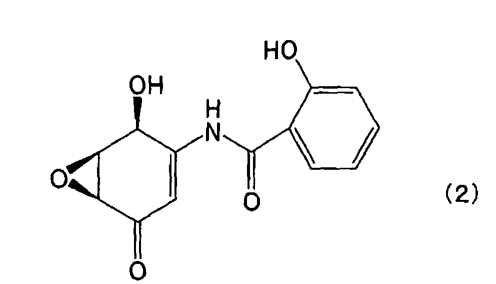

The therapeutic agent for a type IV allergic disease according to the present invention contains a compound represented by the general formula (1) or its pharmacologically acceptable salt as an active ingredient.

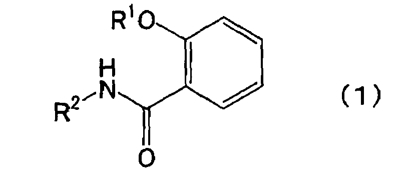

wherein R¹ represents a hydrogen atom or a C2 to C4 alkanoyl group. Examples of the alkanoyl group include an acetyl group, a propionyl group, a butanoyl group, and their isomer groups, of which an acetyl group is particularly preferred.

R² represents a group represented by the following formula (A), (B), (C), (D), (E), (F), or (G):

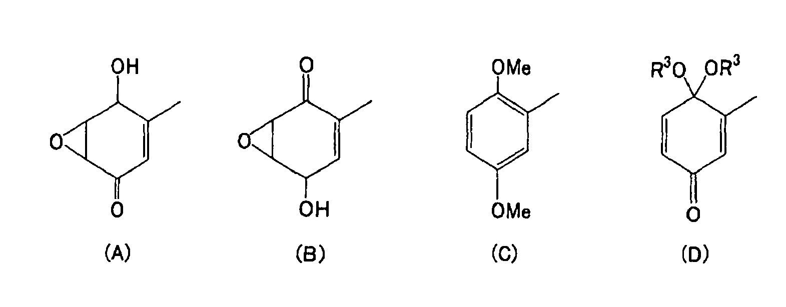

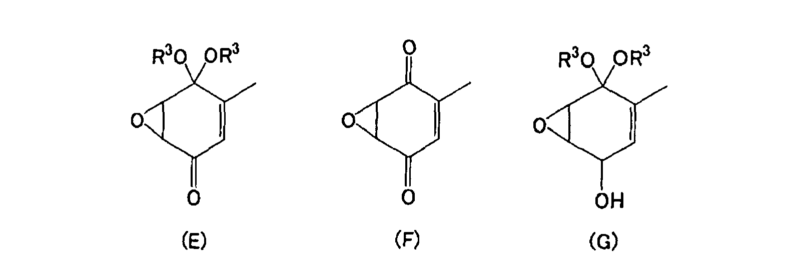

wherein R³ represents a C1 to C4 alkyl group. Examples of the alkyl group include a methyl group, an ethyl group, a propyl group, and butyl group, and their isomer groups, of which a methyl group and an ethyl group are particularly preferred.

Further, the aforementioned compound is preferably (±)-DHM2EQ (the following formula (1a)) or (±)-DHM3EQ (the compound represented by the following formula (1b)) particularly preferably the following formula (2), and most preferably one that substantially does not contain a levorotatory (+) compound.

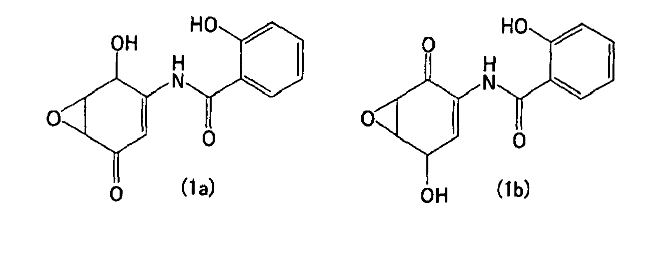

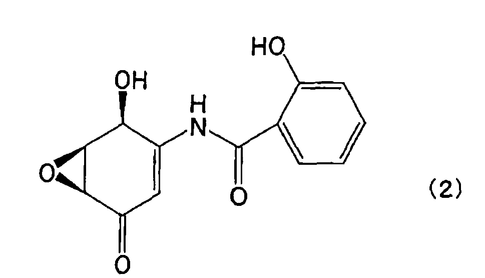

The therapeutic agent for improving severity of an atopic disease associated with pathogenic infection according to the present invention contains a compound represented by the following general formula (1) or its pharmacologically acceptable salt as an active ingredient:

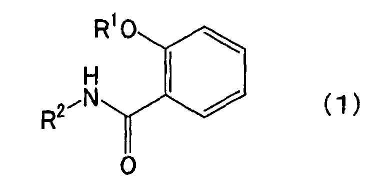

wherein R¹ represents a hydrogen atom or a C2 to C4 alkanoyl group. Examples of the alkanoyl group include an acetyl group, a propionyl group, a butanoyl group, and their isomer groups, of which an acetyl group is particularly preferred.

R² represents a group represented by the following formula (A), (B), (C), (D), (E), (F), or (G):

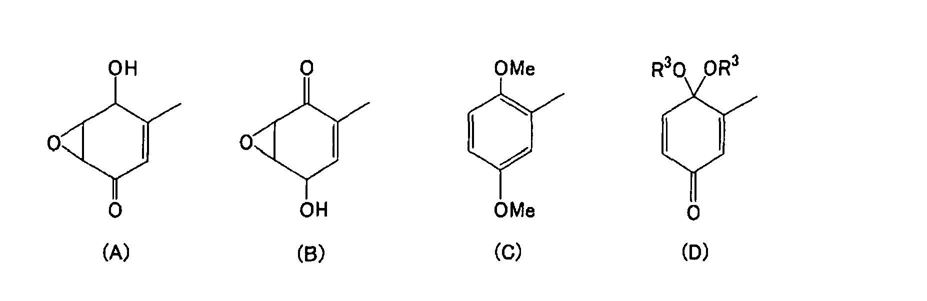

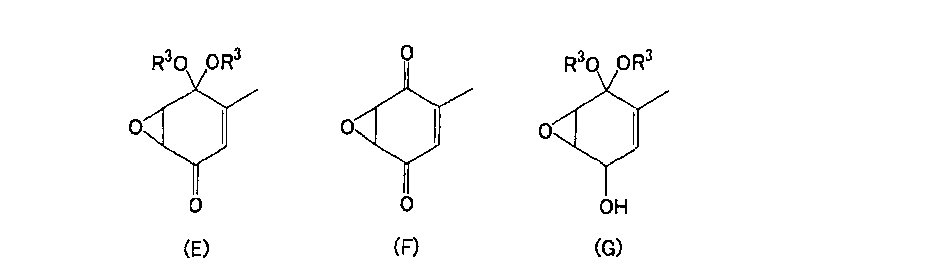

wherein R³ represents a C1 to C4 alkyl group. Examples of the alkyl group include a methyl group, an ethyl group, a propyl group, and butyl group, and their isomer groups, of which a methyl group and an ethyl group are particularly preferred.

Further, the aforementioned compound is preferably (±)-DHM2EQ (the following formula (1a)) or (±)-DHM3EQ (the compound represented by the following formula (1b)), particularly preferably the following formula (2), and most preferably one that substantially does not contain a levorotatory (+) compound.

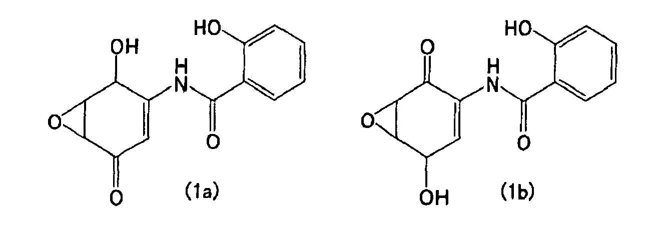

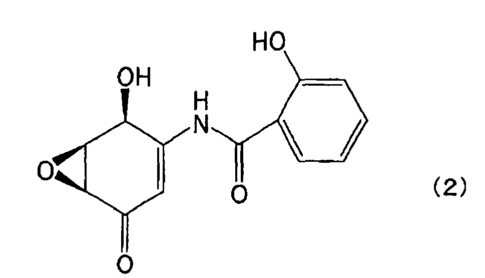

### CROSS REFERENCE TO RELATED APPLICATIONS

This application claims the benefit of priority to Japanese Patent Application No. 2003- 288280, filed on August 6, 2003, which is incorporated herein by reference.

### BRIEF DESCRIPTION OF THE DRAWINGS

FIG. 1 shows the effect of (±)-DHM2EQ to suppress NO production of RAW264.7 cells derived frommacrophages activated by IFN-γ and LPS.

FIG. 2 shows the effect of (±)-DHM2EQ to suppress NO production of RAW264. 7 cells derived frommacrophages activated by IFN-γ and LPS.

FIG. 3 shows the suppressive effect of (±) -DHM2EQ on iNOS production of RAW264.7 cells derived from macrophages activated by LPS.

FIG. 4 shows the suppressive effect of (±)-DHM2EQ on IL-1β production of RAW264.7 cells derived from macrophages activated by LPS.

FIG. 5 shows the suppressive effect of (±)-DHM2EQ on IL-6 production of RAW264.7cells derived frommacrophages activated by LPS.

FIG. 6 shows the suppressive effect of (±)-DHM2EQ on TNF-α production of RAW264.7 cells derived from macrophages activated by IL-1β.

FIG. 7 shows the suppressive effect of (±)-DHM2EQ on NO production of J774.1 cells derived from macrophages activated by IFN-γ and LPS.

FIG. 8 shows the results obtained by examining by Western blotting the suppressive effect of (±)-DHM2EQ on the expression of COX-2 in RAW264.7 cells treated with LPS.

FIG. 9 represents the result showing that (±)-DHM2EQ suppresses the phagocytosis of E. coli by macrophages-derived RAW264.7 cells activated by LPS stimulation.

FIG. 10 represents the result showing that (±) -DHM2EQ suppresses the phagocytosis of E. coli by macrophages-derived RAW264.7 cells activated by IL-1β stimulation.

### BEST MODE FOR CARRYING OUT THE INVENTION

Embodiments of the present invention accomplished based on the above-described knowledge are hereinafter described in detail by giving Examples. Unless otherwise explained, methods described in standard protocols such as J. Sambrook and E. F. Fritsch & T. Maniatis (Ed.),"Molecular Cloning, a Laboratory Manual (3rd edition), Cold Spring Harbor Press and Cold Spring Harbor, New York (2001); F. M. Ausubel, R. Brent, R. E. Kingston, D. D. Moore, J. G. Seidman, J. A. Smith, K. Struhl (Ed.), "Current Protocols in Molecular Biology, "John Wiley & Sons Ltd., or alternatively, modified/changed methods from these are used. When using commercial reagent kits and measuring apparatus, unless otherwise explained, attached protocols to them are used.

The objects, characteristics, and advantages of the present invention as well as the idea thereof are apparent to those skilled in the art from the descriptions given herein. Those skilled in the art can reproduce the present invention easily from the descriptions given herein. It is to be understood that the embodiments and specific examples of the invention described hereinbelow are to be taken as preferred examples of the present invention. These descriptions are for illustrative and explanatory purposes only and are not intended to restrict the invention to these embodiments or examples. It is further apparent to those skilled in the art that various changes and modifications may be made based on the descriptions given herein within the intent and scope of the present invention disclosed herein.

By infection with pathogens, such as viruses and bacteria, monocular spherocytes (e.g., macrophages, monocytes [including peripheral blood monocytes], eosinophils, etc.) are activated to produce inflammatory cytokines (e.g., TNF-α, IL-1β, IL-1α, IL-8, IL-10, etc.), chemokines (MCP-1 and RANTES), iNOS, COX-2, etc. It is known that their production is induced through activation of NF-κB by stimulation of monocular spherocytes by LPS, a component of bacteria, or by macrophage-activating factors, such as IFN-γ, secreted from T cells activated by viral infection.

Thus, on the assumption that (±)-DHM2EQ, an NF-KB activation inhibitor, might inhibit macrophage activation, the inventors examined the effect of (±)-DHM2EQ on activated macrophage cells stimulated with LPS and IFN-γ. As a result, (±)-DHM2EQ inhibited iNOS expression, NO production, IL-1β secretion, and TNF-α secretion, all of which are markers of macrophage activation. The inventors also clarified that (±)-DHM2EQ inhibits COX-2 production and phagocytosis of activated macrophage stimulated with LPS and IL-1β. These findings revealed that (±)-DHM2EQ has an inhibitory effect on macrophage activation. (±)-DHM2EQ is therefore useful as a macrophage activation inhibitor.

Meanwhile, inflammatory cytokines (e.g., TNF-α, IL-1β, IL-1α, IL-8, IL-10, etc.), chemokines (MCP-1 and RANTES), iNOS, COX-2, etc., which are produced with macrophage activation, are known to be the mediators of diseases, such as various infectious diseases, type IV allergic diseases, neurodegenerative diseases including Alzheimer's disease or Parkinson's disease and multiple sclerosis, neurodegenerative diseases as a sequela of brain ischemia, immunodeficiency diseases such as AIDS, arteriosclerosis, periodontosis, inflammatory bowel disorders including ulcerative colitis or Crohn disease, type 2 diabetes, tumors such as solid cancer (including tumor progression), respiratory diseases, pulmonary disease, systemic lupus erythematosus (SLE), which is an autoimmune disease; and of severity of atopic diseases associated with bacterial infection (atopic dermatitis, etc.) . Accordingly, (±)-DHM2EQ, which inhibits macrophage activation, is also useful as an agent for preventing, improving, or treating above-mentioned diseases resulting from macrophage activation and severity of atopic diseases associated with pathogenic infection.

It should be noted that the aforementioned infectious diseases are not limited to any specific one as long as they result from infection with pathogens, such as bacteria, viruses and parasites. Examples of bacteria include, for example, Gram-negative bacteria and Gram-positive bacteria. Examples of viruses include, for example, human T-cell lymphotropic virus type 1 (HTLV-1), influenza viruses, AIDS virus (HIV: human immunodeficiency virus), herpesviruses (e.g., Epstein-Barr virus: EB virus), cytomegalovirus, hepatitis B virus, measles virus, human parvovirus, cronavirus, severe acute respiratory syndrome (SARS) virus, respiratory syncytial (RS) virus, nipah virus, hepatitis C virus, pneumovirus, adenoviruses, Coxsackie virus, etc.

It is known that macrophages, induced by iNOS, produce large amounts and high concentrations of NO. Accordingly, (±)-DHM2EQ is also useful as an agent for preventing, improving, or treating diseases resulting from excessive NO such as, for example, tumors (including metastasis), arteriosclerosis, infections (e.g., AIDS and Helicobacter pylori infection), sepsis, rheumatoid arthritis, multiple sclerosis, dermatitis, periodontosis, asthma, inflammation, heart transplant rejection, Alzheimer's disease, myocardial infarction, autoimmune diseases (e.g. type 1 diabetes), etc.

In addition, it is known that inflammatory cytokines, such as TNF-α, inhibit differentiation of myoblasts into muscle cells (FASEBJ. 15, 1169- 80, 2001) and thereby cause muscular dystrophies including the Duchenne type. Therefore, (±) -DHM2EQ is also useful as an agent for preventing, improving, or treating muscular dystrophies etc. resulting from macrophage activation.

Further, (±)-DHM2EQ is also useful as an agent for preventing, improving, or treating hemophagocytic syndrome, a disease occurring in bone marrow, in which red blood cells are destroyed by overactivated macrophages (hemophagocytes); cerebral infarction, in which motor dysfunctions including paralysis are caused by activation of microglia, macrophages in the brain; the intestinal disease (proliferative enteropathy), characterized by microbial infection and aggregation of activated macrophages in the lesion area, etc.

=== Method for Producing Compounds Represented by the General Formula (1)===
The compounds represented by the general formula (1) can be produced according to the synthetic process by Wipf et al. (Synthesis, No. 12, p. 1549-1561, 1995).

One example of the processes for producing compounds represented by the general formula (1) will be illustrated hereinbelow, based on the following reaction schemes.
In the following reaction scheme, R¹represents a hydrogen atom or a C2 to C4 alkanoyl group. Examples of the alkanoyl group include an acetyl group, a propionyl group, a butanoyl group, and their isomer groups, of which an acetyl group is particularly preferred. R³ represents C1 to C4 alkyl group. Examples of the alkyl group include a methyl group, an ethyl group, a propyl group, a butyl group, and their isomer groups, of which a methyl group and an ethyl group are preferred, and particularly preferred is a methyl group. X represents a halogen atom. Examples of the halogen atom include fluorine, chlorine, bromine, and iodine atoms, of which chlorine and bromine atoms are preferred, and particularly preferred is a chlorine atom.

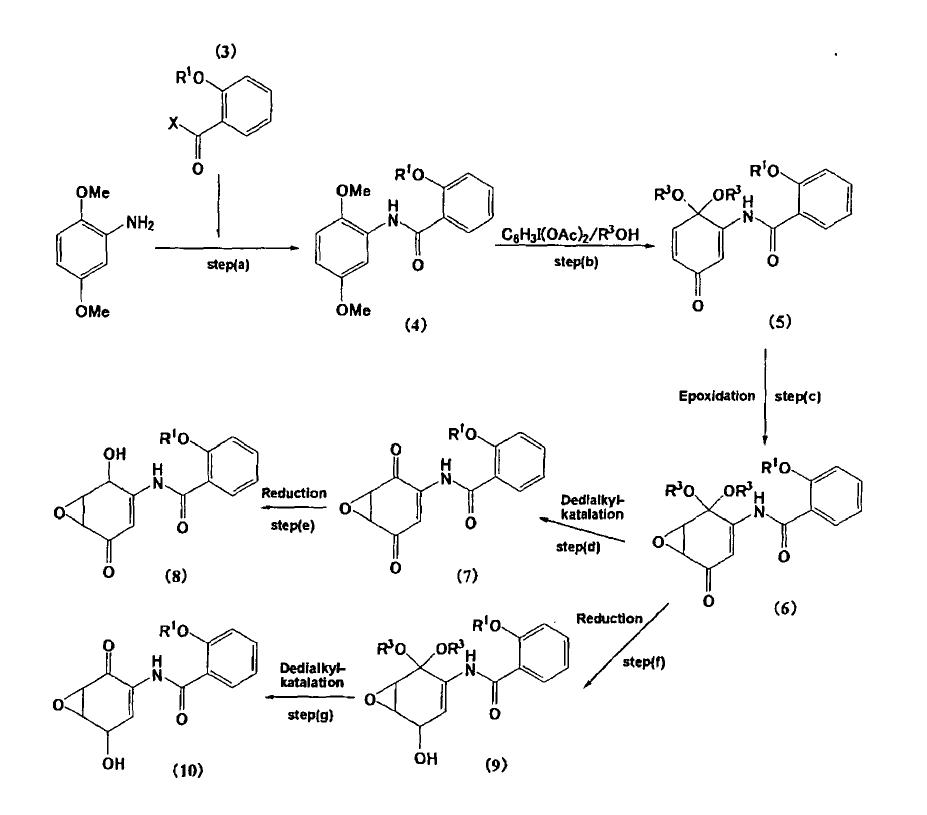

### Step a: Preparation of N-(2-alkanoylbenzoyl)-2,5-dimethoxyaniline

2, 5-Dimethoxyaniline is dissolved in a solvent (pyridine, etc.), to which is added and ethyl acetate solution of O-alkanoylsalicyloyl halide of formula (3) at -78°C to 50°C, preferably under ice cooling, and the mixture is reacted while stirring. After stopping the reaction by addition of water, ethyl acetate is added to the reaction mixture, which then is sequentially washed with hydrochloric acid, water, a sodium hydrogencarbonate solution and water. After drying, the organic layer is concentrated under reduced pressure and dried under vacuum to obtain an N-(2-alkanoylbenzoyl)-2,5-dimethoxyaniline compound represented by formula (4). The compound can be used in the next step without purification.

### Step b: Preparation of 3-(O-alkanoylsalicyloyl) amino-4,4-dialkoxy-2,5-cyclohexadienone

The compound of formula (4) obtained as described above is dissolved in a solvent such as methanol, to which is added diacetoxyiodobenzene at -20°C to 50°C, preferably under ice cooling and the mixture is reacted at room temperature while stirring. After concentration under reduced pressure, ethyl acetate is added and the reaction mixture is washed with sodium hydrogencarbonate solution and saline. Then, the solvent is concentrated under reduced pressure and the obtained residue is purified by column chromatography to obtain 3-(O-alkanoylsalicyloyl)
amino-4,4-dialkoxy-2,5-cyclohexadienone represented by formula (5).

### Step c: Preparation of 5,6-epxoy-4,4-dialkoxy-3-salicyloylamino-2-cyclohexenone

The compound of formula (5) is dissolved in a solvent (tetrahydrofuran, methanol, etc.), to which are added hydrogen peroxide water and sodium hydroxide at -20°C to 50°C, preferably under ice cooling, and the mixture is reacted while stirring. Ethyl acetate is added to the reaction mixture, which is sequentially washed with hydrochloric solution, aqueous sodium thiosulfate solution, and saline. After drying in the air, the reaction mixture is dried under vacuum. In order to remove the residual starting compound, the residue is dissolved in acetone, p-toluenesulfonic acid is added and the starting compound is degraded by stirring at room temperature. Ethyl acetate is added to the residue obtained by distilling off methanol under reduced pressure, and the solution is washed with water. The residue obtained by drying the ethyl acetate layer is purified by column chromatography to obtain 5,6-epxoy-4,9-dialkoxy-3-salicyloylamino-2-cyclohexenone compound represented by formula (6).

### Step d: Preparation of 5, 6-epoxy-2-salicyloylamino-2-cyclohexen-1,4-dione

The compound of formula (6) is dissolved in a solvent (methylene chloride, etc.), an inorganic acid or organic acid (trifluoroboron diethyl ether complex, etc.) is added under ice cooling, and the mixture is reacted while stirring. A solvent (ethyl acetate, etc.) is added to the reaction mixture, which is washed with water. After concentrating the ethyl acetate layer, the obtained residue is washed with methanol to obtain 5,6-epoxy-2-salicyloylamino-2-cyclohexen-1,4-dione represented by formula (7).

### Step e: Preparation of 5,6-epoxy-4-hydroxy-3-salicyloylamino-2-cyclohexenone

The compound of formula (7) is suspended in a solvent (methanol, ethanol, THF, etc.), to which is added a reducing agent (sodium borohydride, etc.) at -78°C to 50° C, preferably under ice cooling. A solvent (ethyl acetate, methylene chloride, etc.) is added to the reaction mixture, which is sequentially washed with hydrochloric acid and water. After drying, the solvent layer is concentrated under reduced pressure, suspended, stirred and washed with methanol to obtain 5,6-epoxy-4-hydroxy-3-salicyloylamino-2-cyclohexenone represented by formula (8).

### Step f: Preparation of 3, 3-dialkoxy-4, 5-epoxy-6-hydroxy-2-salicyloylamino-cyclohexene

The compound of formula (6) is dissolved in a mixed solution of a solvent such as methanol and sodium hydrogen carbonate solution, a reducing agent (sodium borohydride, etc.) is added at -78° C to 50°C, preferably under ice cooling, and the mixture is reacted while stirring. A solvent (ethyl acetate, etc.) is added to the reaction mixture, which is sequentially washed with hydrochloric acid and water. After drying, the solvent layer is concentrated under reduced pressure, dried under vacuum and purified by column chromatography to obtain 3,3-dialkoxy-4,5-epoxy-6-hydroxy-2-salicyloylamide-cyclohex ene represented by formula (9)

### Step g: Preparation of 5,6-epoxy-4-hydroxy-2-salicyloylamino-2-cyclohexenone

The compound of formula (9) is dissolved in a solvent (acetone, etc), p-toluenesulfonic acid is added to the solution, which is then reacted at room temperature while stirring. A solvent (ethyl acetate, etc.) is added to the reaction mixture, which is washed with water. The solvent layer is dried, concentrated under reduced pressure and purified to obtain 5,6-epoxy-4-hydroxy-2-salicyloylamino-2-cyclohexenone represented by formula (10).

It should be noted that (±) -DHM2EQ (formula (1a)) and (±)-DHM3EQ (formula (1b)) can be produced by using O-acetylsalicyloyl chloride and methanol as O-alkanoylsalicyloyl halide of formula (3) and as a solvent for dissolving a compound of formula (4) in step (b), respectively.

=== Preparation of compounds represented by formula (2)
In recent years, the inventors have found that (-) -DHM2EQ has a stronger inhibitory effect on NF-KB activation than (±)-DHM2EQ. Accordingly, (-)-DHM2EQ is considered to have a stronger inhibitory effect on macrophage activation than (±)-DHM2EQ. This suggests that (-)-DHM2EQ with stronger inhibitory effect on macrophage activation than (±)-DHM2EQ is more useful as an agent for preventing, improving, or treating severity of disease resulting from macrophage activation, diseases induced by excessive NO, and atopic diseases associated with pathogenic infection. In addition, it can be expected that (-) -DHM2EQ has fewer side effects than (±) -DHM2EQ. Preparation of (-)-DHM2EQ (formula (2)) is explained hereinbelow.

A compound ((-)-DHM2EQ) represented by formula (2) can be obtained, as shown in the following reaction formula, by once protecting the phenolic hydroxyl group of (±) - DHM2EQ (formula (1a)) with a silyl group, resolving the resulting compound on a chiral column into a compound represented by formula (12) and a compound represented by formula (13), and then deprotecting the compound represented by formula (12) (Bioorg.Med.Chem.Lett.10,865- 869, 2000). It should be noted that by deprotecting the compound represented by formula (13) as well in the same manner as above, it is also possible to obtain a compound ((+)-DHM2EQ) represented by formula (14).

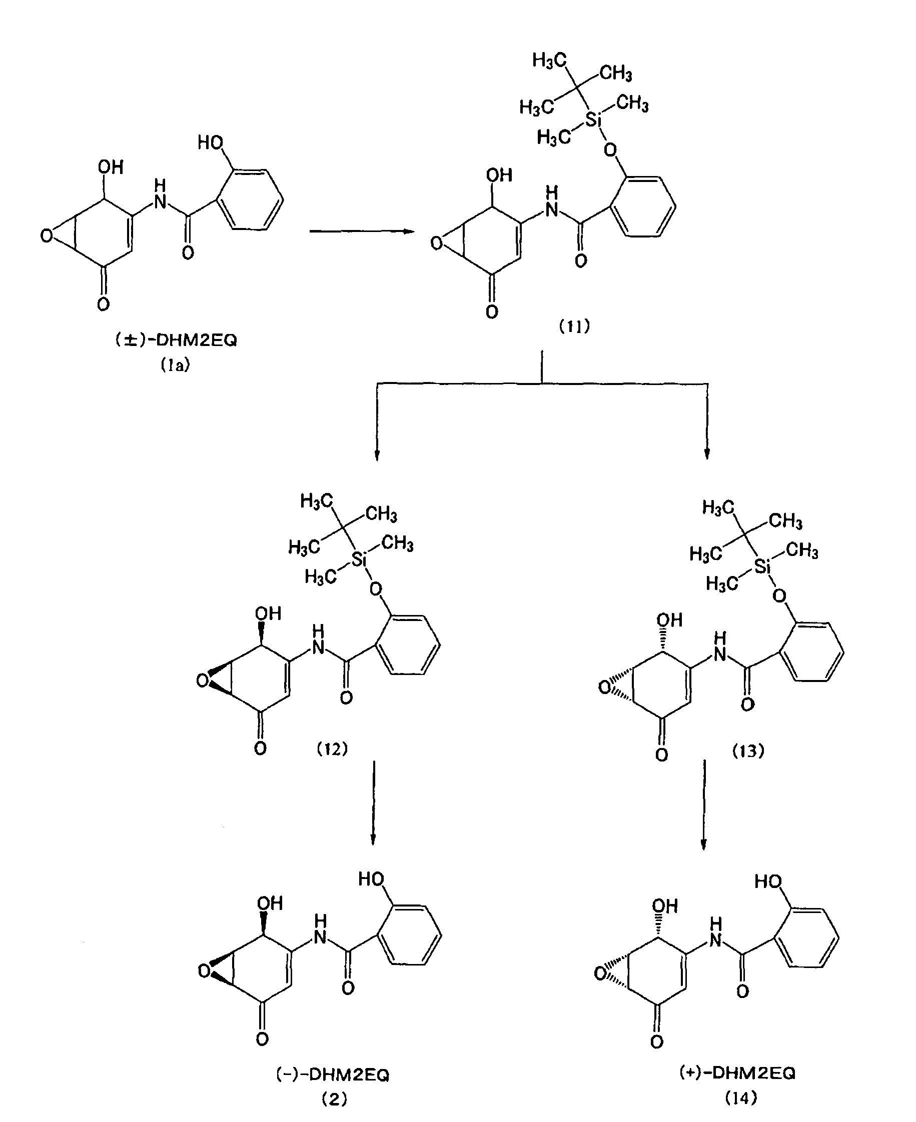

Further, a compound ((±)-DM42EQ) represented by formula (2)

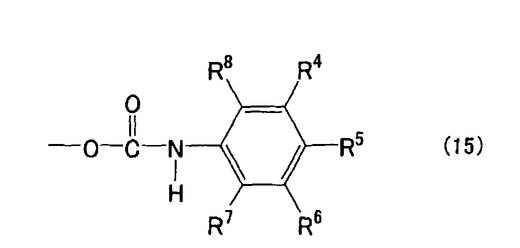

can also be obtained by being directly resolved into (-)-DHM2EQ (dextrorotatory DHM2EQ) and (+)-DHM2EQ (levorotatory-DHM2EQ) using a resolving agent containing as an active ingredient a polysaccharide aromatic carbamate derivative substituted with a group represented by general formula (15).

Examples of polysaccharide aromatic carbamate derivatives include the polysaccharide aromatic carbamate derivatives in which the substituents (-R⁴, -R⁵, -R⁶, -R⁷, and -R⁸) in the general formula (15) are composed of a hydrogen atom, an alkyl group having 1 to 8 carbon atoms, an alkoxy group having 1 to 8 carbon atoms, an aromatic group having 6 to 14 carbon atoms, or a halogen atom, etc. Preferred are amylose tris (3, 5-dimethylphenylcarbamate), cellulose tris (3, 5-dimethylphenylcarbamate), etc., of which amylose tris (3, 5-dimethylphenylcarbamate) is particularly preferred. Examples of the aforementioned halogen atom include, for example, a fluorine atom, a chlorine atom, a bromine atom, an iodine atom, etc.

The aforementioned optical resolution of (±)-DHM2EQ is preferably performed by high performance liquid chromatography using an optically active column packed with a resolving agent containing as an active ingredient a polysaccharide aromatic carbamate derivative substituted with a group represented with the general formula (15), for example, a DAICEL CHIRALPAK AD column (10 mm i.d. x 250 mm) . As a mobile phase, for example, 0.1 to 0.9 v/v% acetic acid in methanol can be used.

The optical density of (-)-DHM2EQ and (+)-DHM2EQ thus obtained can be determined by measuring each optical rotation with JASCODIP-3 60 polarimeter ((-)-DHM2EQ: [α]²⁰_{D} -24 1° (c0.1 MeOH), (+) -DHM2EQ: [α]²⁰_{D} +239° (c0.1 MeOH)).

The macrophage activation inhibitor according to the present is not limited to any specific one as long as it contains a compound represented by the general formula (1) or its pharmacologically acceptable salt as an active ingredient. Preferred are (±)-DHM2EQ and (±)-DHM3EQ, with (-)-DHM2EQ being particularly preferred. In addition, the therapeutic agent for infectious diseases, therapeutic agent for type IV allergic diseases, and therapeutic agent for improving severity of atopic diseases associated with pathogenic infection according to the present invention are also not limited to any specific ones as long as they contain a compound represented by the following general formula (1) or its pharmacologically acceptable salt as an active ingredient. Preferred are (±)-DHM2EQ and (±)-DHM3EQ, with (-)-DHM2EQ being particularly preferred.

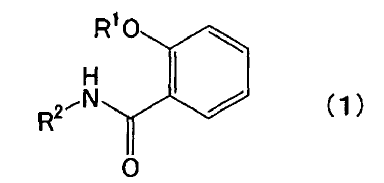

wherein R¹ represents a hydrogen atom or a C2 to C4 alkanoyl group. Examples of the alkanoyl group include an acetyl group, a propionyl group, a butanoyl group, and their isomer groups, of which an acetyl group is particularly preferred.

R² represents a group represented by the following formula (A), (B), (C), (D), (E), (F), or (G):

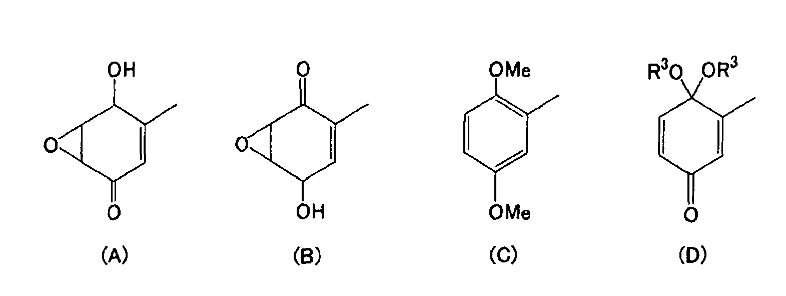

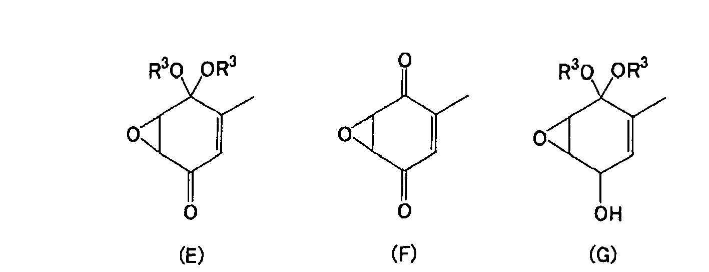

wherein R³ represents a C1 to C4 alkyl group. Examples of the alkyl group include a methyl group, an ethyl group, a propyl group, and butyl group, and their isomer groups, of which a methyl group and an ethyl group are particularly preferred.

In addition, as the macrophage activation inhibitor according to the present invention, one that does not contain (+)-DHM2EQ but contains (-)-DHM2EQ or its pharmacologically acceptable salt as an active ingredient is most preferred. Likewise, as the therapeutic agent for infectious diseases, therapeutic agent for type IV allergic diseases, and therapeutic agent for improving severity of atopic diseases associated with pathogenic infection according to the present invention, one that does not contain (+)-DHM2EQ but contains (-)-DHM2EQ or its pharmacologically acceptable salt as an active ingredient is most preferred.

### EXAMPLES

Examples according to the present invention will be described in detail hereinbelow.

### EXAMPLE 1

### Suppression of NO Production in Macrophage-derived RAW264.7 Cells by (±)-DHM2EQ

It is known that, in macrophages, the expression of iNOS is induced by lipopolysaccharides such as LPS and interferons such as IFN-γ, thereby triggering NO production. Thus, by using macrophage-derived cultured cells, the influence of (±) -DHM2EQ on NO production when LPS and IFN-γ were used in combination with (±)-DHM2EQ was examined.

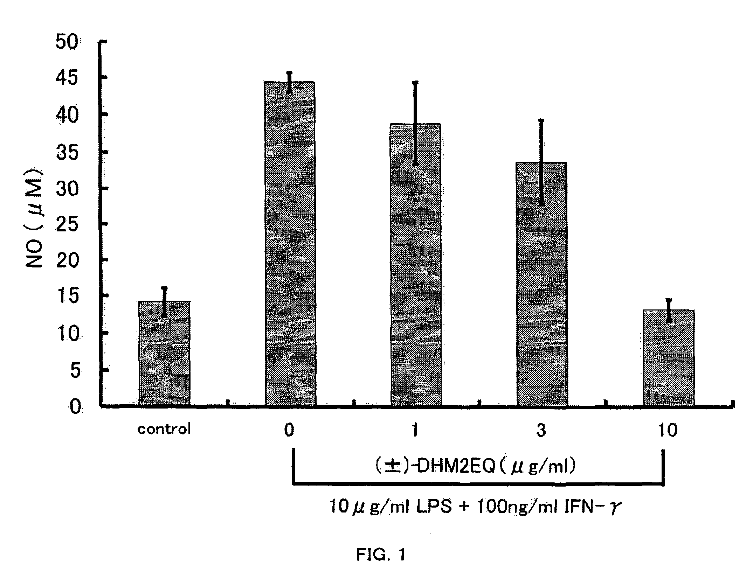

Since NO produced is released out of cells and mixed in the medium, being present as NO₂⁻, the amount of NO produced was determined by developing a color reaction and measuring optical density (OD₅₇₀) after the addition of the Griess reagent to the a medium. Then, each amount of NO produced when (±) -DHM2EQ was applied at different concentrations was compared as a relative value by taking OD₅₇₀ of the sample without the addition of macrophage activating factor as 100%. The results are shown in FIG. 1.

In RAW264.7 cells, when LPS and IFN-γ were added, approximately 3-fold increase in NO production was observed without (±)-DHM2EQ treatment as compared with the control. Then, by treating with (±) -DHM2EQ, NO production was suppressed depending on the concentration of (±)-DHM2EQ added. The NO production was suppressed almost to the control level by adding 10 µg/ml (±) -DHM2EQ.

### METHOD OF EXPERIMENT

A cell suspension prepared at 4 x 10⁵ cells/ml was plated at 200 µl/well in 96-well plates (Costar:Acton, MA). (±)-DHM2EQ was diluted at 0, 1, 3, and 10 µg /ml and added to each well of the plates. After 2-hour incubation under the condition of 37°C in 5% CO₂, IFN-γ (final concentration: 100 ng/ml) and LPS (final concentration: 10 µg/ml) were added to the wells and then the plates were incubated for 18 hours. 100 µl of the Griess reagent was added to each of the cell suspensions and allowed to react for 10 min, and then OD₅₇₀ was measured with a microplate reader.

### EXAMPLE 2

### Suppression of NO Production in Macrophage-derived RAW264.7 Cells by (±)-DHM2EQ

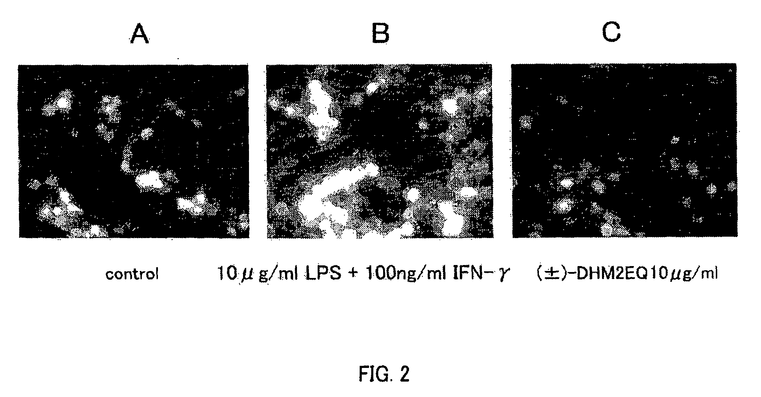

Further, to observe suppression of NO production in situ in macrophage-derived RAW264. 7 cells by (±)-DHM2EQ when LPS and IFN-γ are used in combination with (±)-DHM2EQ, intracellular NO was directly observed using diaminofluorescein-2 diacetate (DAF-2DA; Daiich Pure Chemicals) . The results are shown in FIG. 2.

Compared with the control (A) to which neither LPS nor IFN-γ was added, NO production was enhanced in the cells to which LPS and IFN-γ were added but (±)-DHM2EQ was not added, whereas NO production was suppressed to the control level in the cells to which both LPS and IFN-γ plus 10 µg/ml (±) -DHM2EQ were added. This indicates that NO production is not suppressed in specific cells but suppressed uniformly in cultured cells as a whole.

### METHOD OF EXPERIMENT

500 µl of RAW264.7 cells were first plated in each chamber on chamber slides at a concentration of 6 x 10⁵ cells/ml. After 1 day incubation under the condition of 37°C in 5% CO₂, (±)-DHM2EQ was added, followed by 2-hour incubation. Subsequently, IFN-γ (final concentration: 100 ng/ml) and LPS (final concentration: 10 µg/ml) were added, followed by 6-hour incubation. Then, the medium was changed to DAF-2DA-containing phosphate buffered saline (PBS; 8 g/l NaCl, 0.2 g/l KCl, 0.916 g/l Na₂HPO₄, 0.2 g/l KH₂PO₄), followed by further 2-hour incubation. The fluorescence images were then observed with a fluorescence microscope.

### EXAMPLE 3

### Suppression of iNOS Expression in Macrophage-derived RAw264.7 Cells by (±)-DHM2EQ

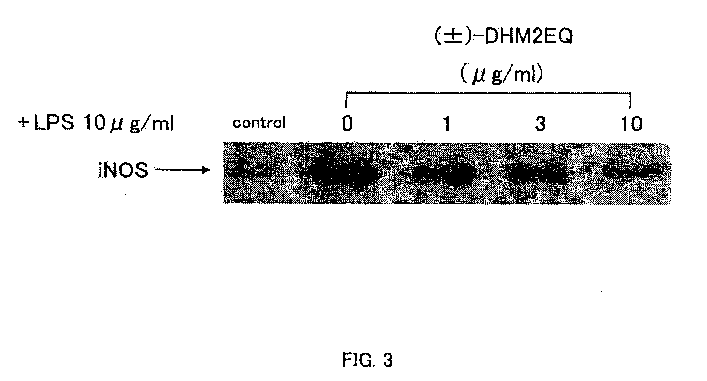

Examples 1 and 2 revealed that (±) -DHM2EQ suppresses NO production of activated RAW264.7 cells. Since NO is produced by the enzyme iNOS within cells, it was examined whether this suppression results from suppression of iNOS expression. As shown in FIG. 3, it was revealed that (±) -DHM2EQ suppresses the expression of iNOS depending on its treatment concentration.

### METHOD OF EXPERIMENT

Cells were plated in 100 mm dishes at 4 x 10⁵ cells/ml, incubated overnight, and then (±)-DHM2EQ was added at final concentrations of 0, 1, 3, and 10 µg/ml. After 2 hours, LPS (final concentration: 10 µg/ml) was added to stimulate for 24 hours. Then, cell suspension was collected and centrifuged, and cells were lysed in 65 µl of prepared lysis buffer (50 mM Tris-HCl: pH 8.0, 1% NP-40, 20 mM EDTA, 100 mM NaVO₄, 0.1 mg/ml leupeptin, 1 mM PMSF). Then, the supernatant of the cell lysate was isolated, each protein concentration was measured, and the predetermined mass of protein was boiled as a sample. This sample was subjected to SDS-PAGE and the proteins on the gel were transferred to a nitrocellulose membrane, which was then reacted with anti-iNOS antibody (Santa Cruz) followed by sheep anti-rabbit IgG antibody (Amersham). Subsequently, color was developed by the ECL detection system, and the signal was detected by exposing the membrane to the film (Fuji Photo Film) .

### EXAMPLE 4

### Suppression of IL-1β Secretion from Macrophage-derived RAW264. 7 Cells by (±)-DHM2EQ

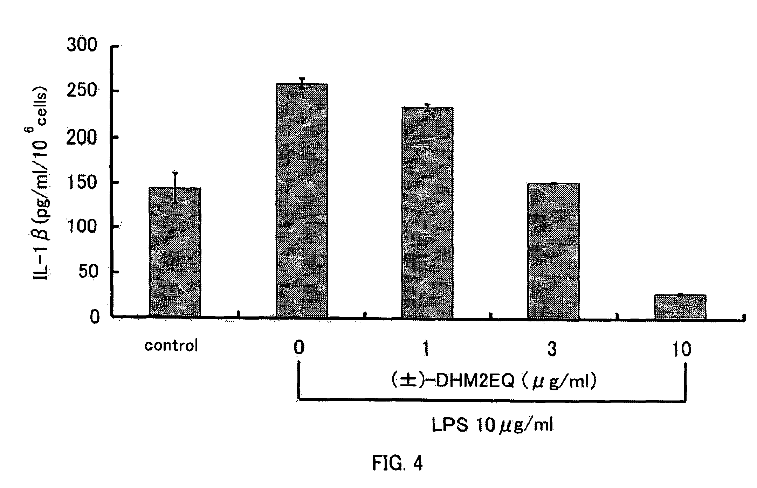

Next, the effect of (±)-DHM2EQ on IL-1β secretion, which is another marker of macrophage activation in macrophage-derived RAW264.7 cells activated by (±) -DHM2EQ, was examined. As a result, as shown in FIG. 4, it was revealed that (±)-DHM2EQ suppresses the expression of IL-1β depending on its treatment concentration.

### METHOD OF EXPERIMENT

500 µl of RAW264.7 cells were plated in each well at a concentration of 4 x 10⁵ cells/ml, and the plates were incubated at 37°C for 1-day. (±)-DHM2EQ (final concentrations: 0, 1, 3, and 10 µg/ml) was then added followed by 2-hour incubation and then LPS (final concentration: 10 µg/ml) was added followed by 24-hour stimulation. After the stimulation, the cell supernatant was recovered (500 µ/l each), and centrifuged at 15000 rpm for 2 min, and the insoluble fractions were removed; the samples were thus obtained.

The amount of IL-1β secretion was examined using mouse IL-1β ELISA assay kit (R&D Systems; Minneapolis, U.S.). First, 50 µl of Reaction Buffer was added to 96-well microplates, on which mouse IL-1β antibody (polyclonal antibody) had been fixed, and 50 µl of the aforementioned sample were applied to each well of the plates. The plate was tapped gently and then left for the reaction at room temperature for 2 hours, being covered with the seal supplied with the kit to avoid exposure to light. Next, 400 µl of wash buffer was applied to each well, the supernatant was discarded by aspiration. This procedure was repeated 5 times and the plate was tapped repeatedly on a paper towel for complete removal of the supernatant. Next, 100 µl of mouse IL-1β secondary antibody conjugated with horse radish peroxydase (HRP) was added to each well. After 2-hour incubation at room temperature, the wells were washed five times in the same manner as described above. Subsequently, 100 µl of coloring solution was added to each well, and the plates were incubated for 30 min. Subsequently, 100 µl of stopping solution was added and the OD value at 450 nm was measured using a microplate reader. Then, the concentration of IL-1β was determined from the calibration curve constructed using mouseIL-1β standard supplied with mouse IL-1β ELISA assay kit.

### EXAMPLE 5

### Suppression of IL-6 Secretion from Macrophage-derived RAW264.7 Cells by (±)-DHM2EQ

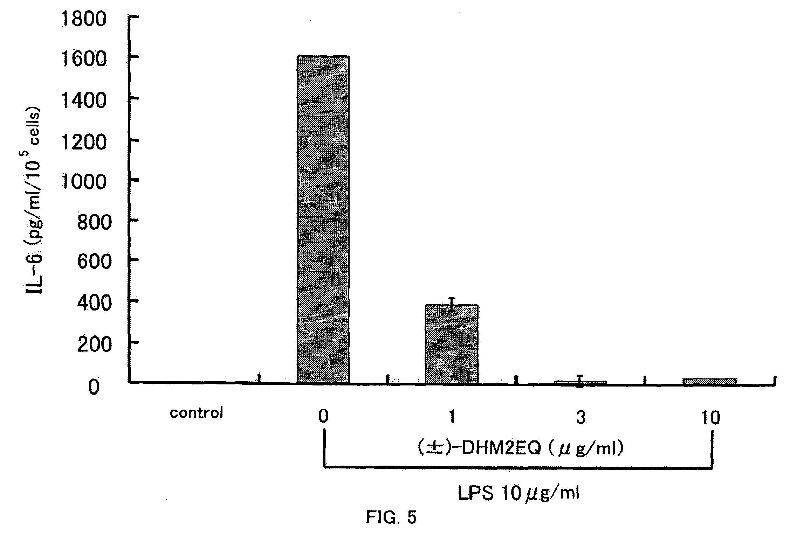

The influence of (±)-DHM2EQ on IL-6 secretion, another marker of macrophage activation in LPS-activated macrophages was examined using IL-6 ELISA assay kit (Techne; Minneapolis, U.S.) in the same manner as in the method of experiment in Example 4. It should be noted that the stimulation of RAW264. 7 cells with LPS (final concentration: 10µg/ml) was performed for 24 hours. In addition, the calibration curve was constructed by IL-6 standard supplied with the IL-6 ELISA assay kit. As a result, as shown in FIG. 5, it was confirmed that (±) -DHM2EQ suppresses IL-6 production depending on its treatment concentration.

### Example 6

### Suppression of TNF-α Secretion from RAW264. 7 Cells Derived from Macrophages Activated by IL-1βStimulation

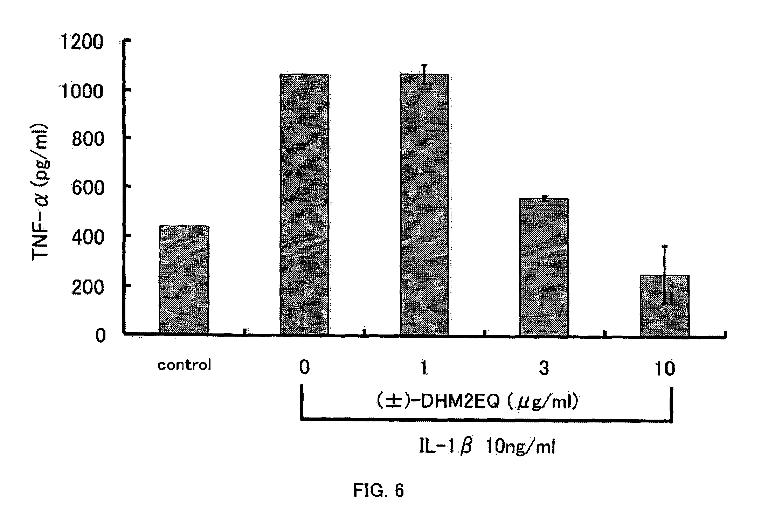

Next, the effect of (±)-DHM2EQ on IL-6 secretion, another marker of macrophage activation in RAW264.7 cells derived from macrophages activated by IL-1β stimulation was examined in the same manner as in the method of experiment in Example 5. It should be noted that in this example, RAW264.7 cells were stimulated for 6 hours with IL-1β (final concentration: 10 ng/ml) in place of LPS. As a result, as shown in FIG. 6, it was confirmed that (±)-DHM2EQ suppresses TNF-α production depending on its treatment concentration.

### EXAMPLE 7

### Suppression of NO Production from Macrophage-derived J774.1 Cells by (±)-DHM2EQ

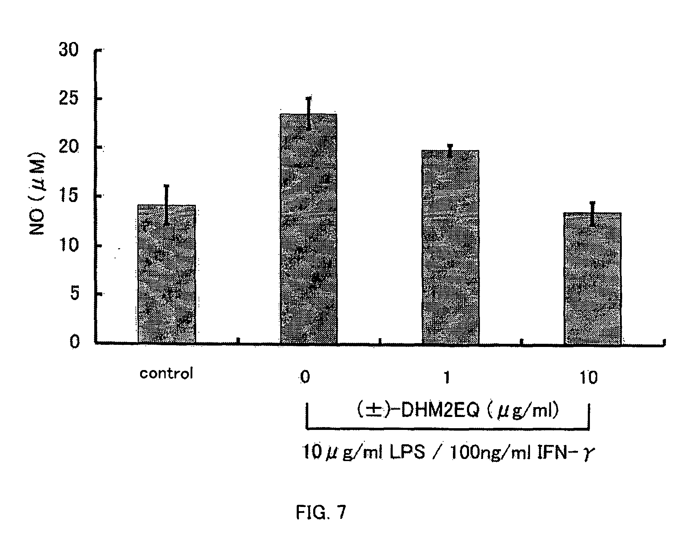

The effect of (±)-DHM2EQ on NO production in another activated macrophage-derived cell line, J774.1, was examined in the same manner as described in Example 1. As a result, as shown in FIG. 7, about 1.5-fold increase in NO production was observed in cells without (±) -DHM2EQ treatment as compared with the control cells (without activation treatment), indicating that NO production is suppressed depending on (±)-DHM2EQ treatment concentration. In 10 µg/ml (±) -DHM2EQ-treated cells, NO production was suppressed to the control level. In conclusion, the suppressive effect of (±)-DHM2EQ on NO production in cells is similarly observed in other macrophages, not being limited to RAW264.7.

### EXAMPLE 8

### Inhibitory Effect of (±)-DHM2EQ on COX-2 Activation

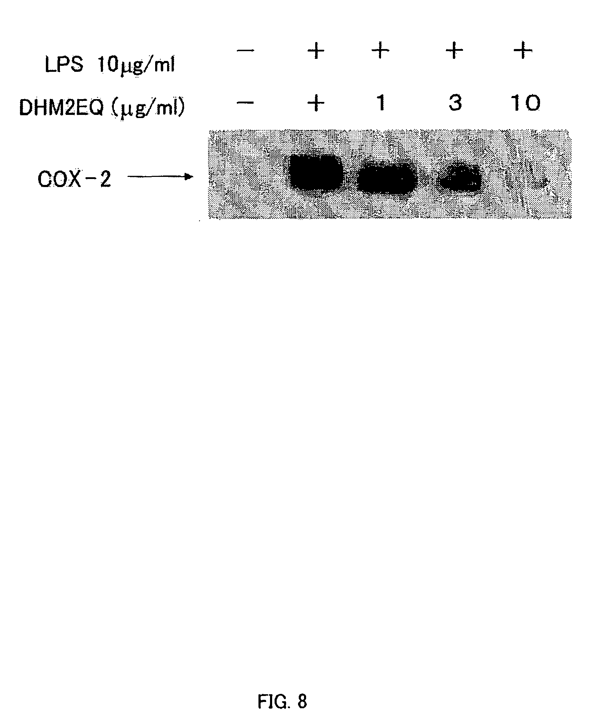

It is known that macrophages activated by inflammatory stimuli and growth factor produces COX-2, a prostaglandin synthase, which is responsible for various pathological conditions such as inflammation, cancer cell proliferation, cancer development, angiogenesis, tumor metastasis, etc. Further, it has recently been known that, in systemic lupus erythematosus (SLE), which is an autoimmune disease, self-reactive T cells acquire resistance to apoptosis with increasing COX-2 expression (Nature Medicine 10, 411-415, 2004) . Thus, on the assumption that use of (±)-DHM2EQ might make it possible to inhibit macrophage activation and thereby to suppress COX-2 production, the effect of (±)-DHM2EQ on COX-2 production in RAW264.7 cells derived from macrophages activated by LPS stimulation was examined. As a result, as shown in FIG. 8, it was found that (±)-DHM2EQ suppresses COX-2 production depending on its treatment concentration. This suggests that (±) -DHM2EQ is also useful as an agent for preventing, improving, or treating diseases caused by COX-2 whose expression is induced with macrophage activation.

### METHOD OF EXPERIMENT

RAW264.7 cells were plated at 4 x 10⁶ cells/dish in a 100 mm dish (Coaster:Acton, MA) containing DMEM medium, and the plates were incubated overnight at 37°C under 5% CO₂. Then, (±) -DHM2EQ (final concentrations: 1, 3, and 10 µg/ml) was added followed by 2-hour incubation and then LPS (final concentration: 10 µg/ml) was added followed by 24-hour stimulation. The cells were then recovered and lysed with 65 µl of lysis buffer (50 mM Tris-HCl: pH 8.0, 1% NP-40, 20 mM EDTA, 100 mM NaVO₄, 0.1 mg/ml leupeptin, 1 mM PMSF) and SDS-PAGE was performed.

Subsequently, Western blotting was performed using a PVDF membrane. Anti-C0X-2 antibody (manufactured by Santa Cruz: diluted by 300 fold) and donkey anti-goat IgG antibody (manufactured by Santa Cruz: diluted by 3000 fold) were used as the primary antibody and second antibody, respectively, and the detection was performed using the enhanced chemiluminescence (ECL).

### EXAMPLE 9

### Inhibitory Effect of (±)-DHM2EQ on Macrophage Phagocytosis

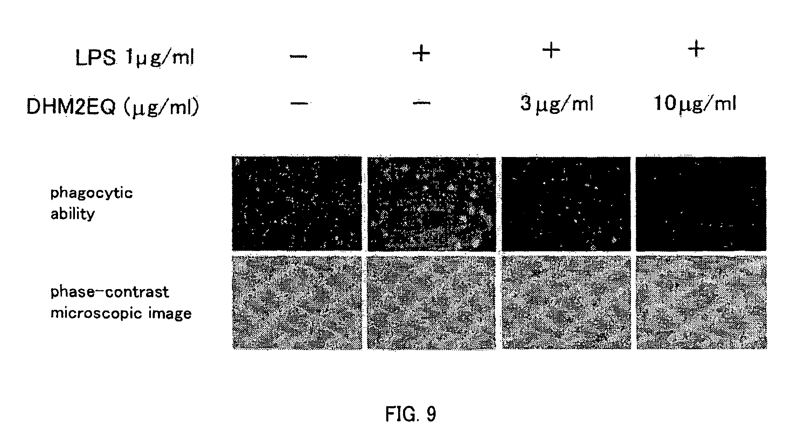

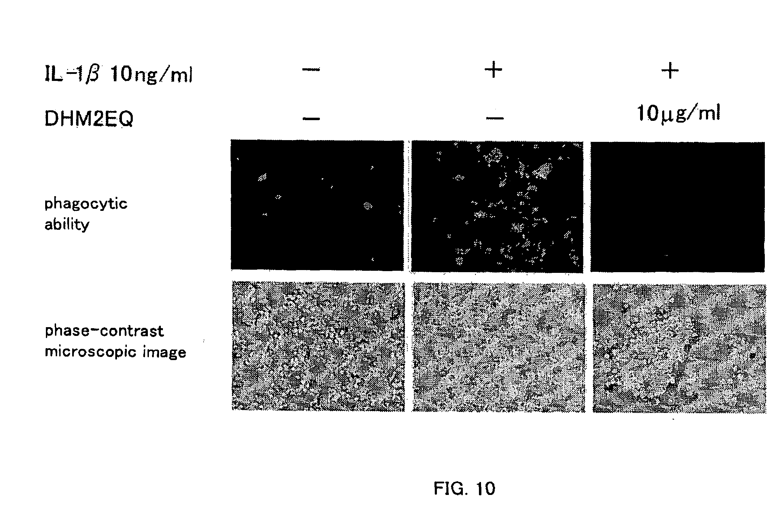

Macrophages contribute to host defense mechanism by, when infected with bacteria, engulfing the whole or part of the bacteria as foreign matter and digesting the foreign matter in the lysosome containing hydrolytic enzymes, etc. This penomenon of engulfing foreign matter, referred to as phagocytosis, is marker of macrophage activation. Thus, by using as a marker phagocytotic ability of RAW264.7 cells derived from macrophages activated by the LPS or IL-1β stimulation, the effect of (±) -DHM2EQ on macrophage activation was examined. As shown in FIG. 9, it was found that the phagocytosis of E. coli by macrophage-derived RAW264.7 cells is markedly enhanced by LPS stimulation, but that, when cells were pretreated with (±)-DHM2EQ, the phagocytosis is suppressed depending on its treatment concentration. Specifically, it was shown that when cells are pretreated with 10 µg/ml (±) -DHM2EQ, the phagocytosis is suppressed almost to the same level as the control without LPS or (±)-DHM2EQ treatment. Likewise, as shown in FIG.10, it was revealed that in RAW264.7 cells derived from macrophages activated by IL-1β stimulation as well, by pretreatment with 10 µg/ml (±)-DHM2EQ, the phagocytic action of E. coli is suppressed almost to the same level as the control without LPS or (±)-DHM2EQ treatment.

### METHOD OF EXPERIMENT

500 µl of RAW264.7 cells were plated in each chamber at a concentration of 4 x 10⁵ cells/ml in chamber slides. After an overnight incubation, (±)-DHM2EQ was added, and the slides were incubated for 2 hours. LPS (final concentration: 1 µg /ml) was then added, and the slides were incubated for 10 mins and then washed with 750 µl of PBS two times. Subsequently, PBS was removed and 250 µl of the solution containing fluorescent-labeled E. coli (Molecular probes, U.S.) (i.e. a solution of fluorescent-labeled E. coli diluted with 5 mg of PBS) was added to each chamber, and the slides were incubated for 2 hours at room temperature the cells were then washed twice in 750 µl of PBS and the phagocytic ability of macrophage-derived RAW264.7 cells were observed under a fluorescence microscope and a phase-contrast microscope. In addition, in the activation using IL-1β, the phagocytic ability of macrophage-derived RAW264.7 cells was examined in the same manner as above, except that IL-1β (final concentration: 10 ng/ml) was added in place of LPS. As shown in FIGS. 9 and 10, since the macrophages stimulated with LPS or IL-1β engulf fluorescent-labeled E. coli, the cells were labeled with fluorescence and their morphology becomes large and flat, whereas the cells not stimulated or treated with DHM2EQ, were small and spherical and their fluorescence was observed outside the cells.

### INDUSTRIAL APPLICABILITY

According to the present invention, macrophage activation inhibitors useful to prevent, improving, or treating diseases resulting from infection with pathogens, such as bacteria and viruses, and immune diseases, such as allergies, can be provided.

## Claims

1. An inhibitor for inhibiting macrophage activation, comprising a compound represented by the following general formula (1) or its pharmacologically acceptable salt as an active ingredient, 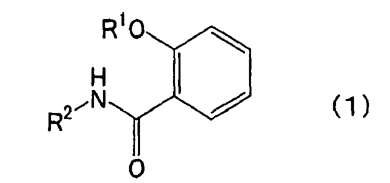 wherein R¹ represents a hydrogen atom or a C2 to C4 alkanoyl group and R² represents a group represented by the following formula (A), (B), (C), (D), (E), (F), or (G): 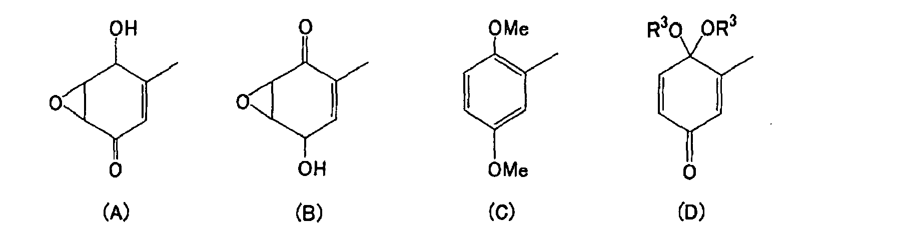 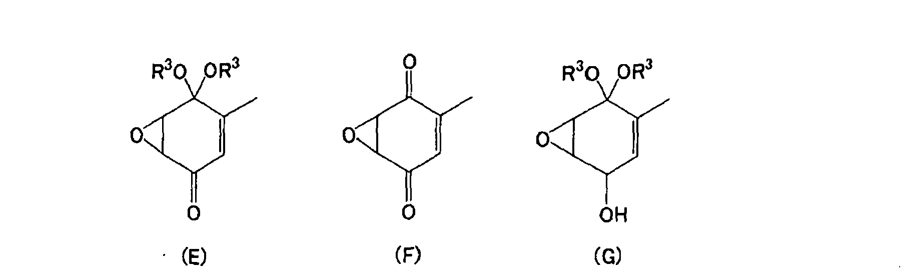 wherein R³ represents a C1 to C4 alkyl group.

2. The inhibitor of claim 1, wherein the compound is the following formula (1a) or formula (1b):

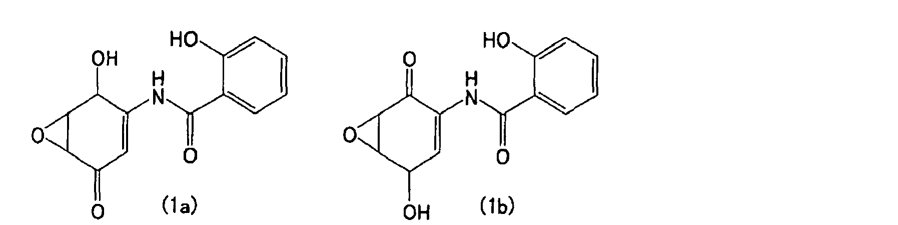

3. The inhibitor of claim 1, wherein the compound is the following formula (2):

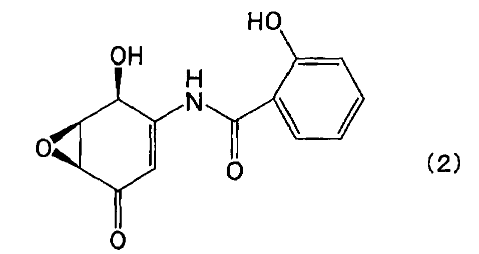

4. The inhibitor of any one of claims 1 to 3, which prevents, improves, or treats a disease resulting from macrophage activation.

5. The inhibitor of claim 4, wherein the disease is an infectious disease.

6. The inhibitor of claim 5, wherein the infectious disease results from infection with a bacterium.

7. The inhibitor of claim 6, wherein the bacterium is a Gram-negative bacterium.

8. The inhibitor of claim 5, wherein the infectious disease results from infection with a virus.

9. The inhibitor of claim 8, wherein the virus is influenza.

10. The inhibitor of claim 4, wherein the disease is a type IV allergic disease.

11. The inhibitor of any one of claims 1 to 3, which prevents, improves, or treats severity of an atopic disease associated with pathogenic infection.

12. The inhibitor of any one of claims 1 to 3, wherein the macrophage activation results from any one factor selected from the group consisting of lipopolysaccharide, IFN-γ, and IL-1β.

13. A therapeutic agent for an infectious disease, comprising a compound represented by the following general formula (1) or its pharmacologically acceptable salt as an active ingredient: 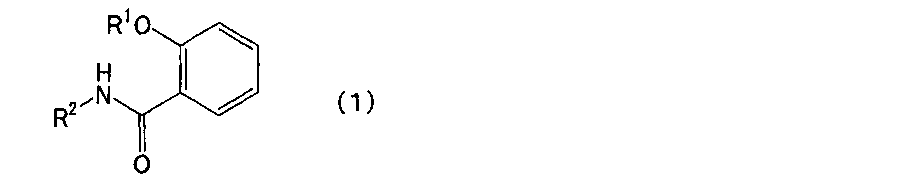 wherein R¹ represents a hydrogen atom or a C2 to C4 alkanoyl group and R² represents a group represented by the following formula (A), (B), (C), (D), (E), (F), or (G): 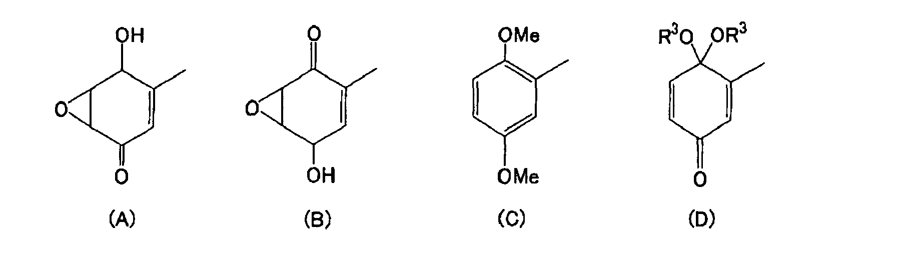 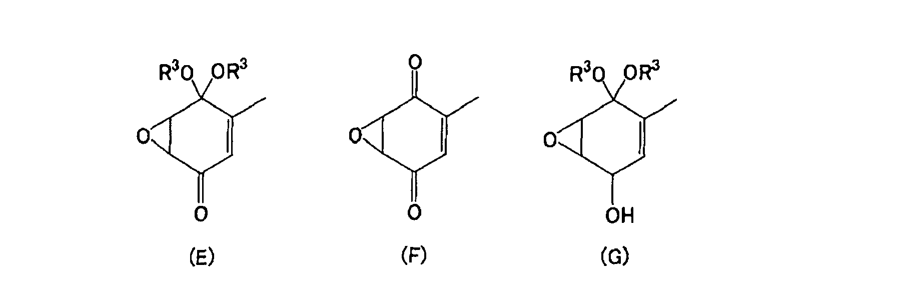 wherein R³ represents a C1 to C4 alkyl group.

14. The therapeutic agent for an infectious disease of claim 13, wherein the compound is the following formula (1a) or formula (1b):

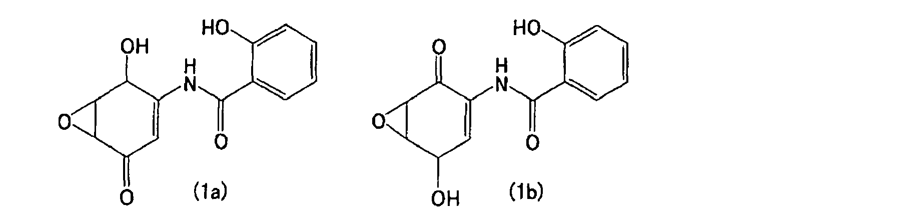

15. The therapeutic agent for an infectious disease of claim 13, wherein the compound is the following formula (2):

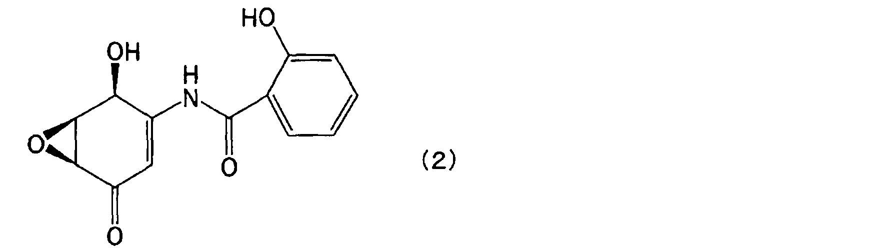

16. A therapeutic agent for a type IV allergic disease, comprising a compound represented by the following general formula (1) or its pharmacologically acceptable salt as an active ingredient: 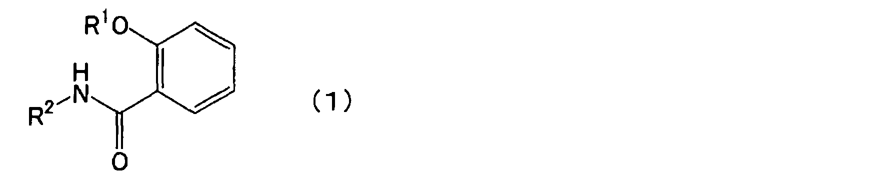 wherein R¹ represents a hydrogen atom or a C2 to C4 alkanoyl group and R² represents a group represented by the following formula (A), (B), (C), (D), (E), (F), or (G): 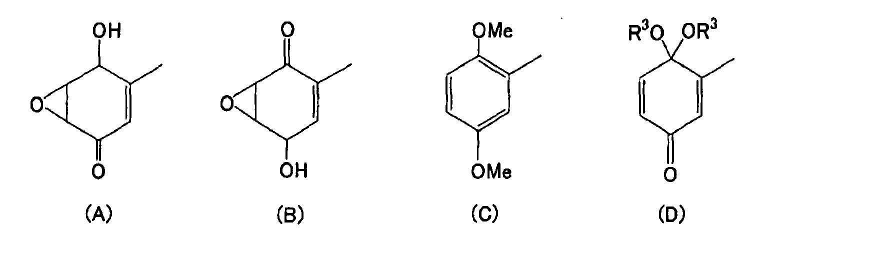 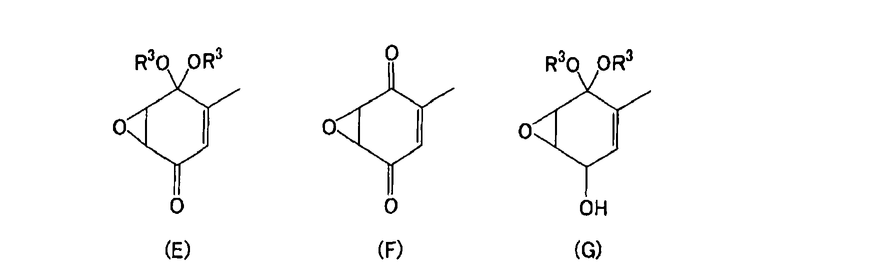 wherein R³ represents a C1 TO C4 alkyl group.

17. The therapeutic agent for a type IV allergic disease of claim 16, wherein the compound is the following formula (1a) or formula (1b):

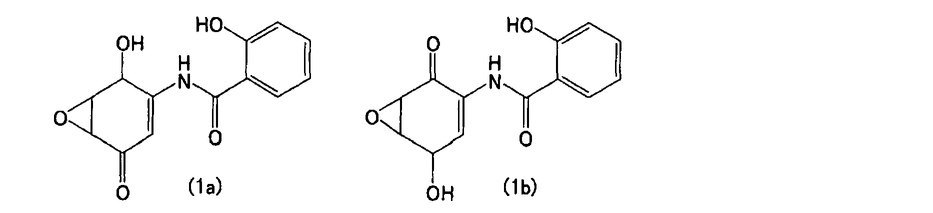

18. The therapeutic agent for a type IV allergic disease of claim 16, wherein the compound is the following formula (2):

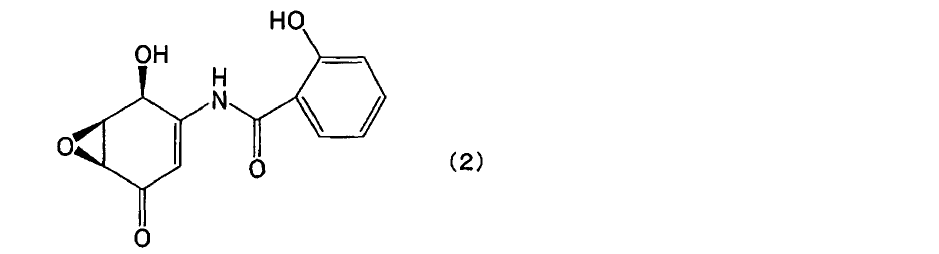

19. A therapeutic agent for improving severity of an atopic disease associated with pathogenic infection, comprising a compound represented by the following general formula (1) or its pharmacologically acceptable salt as an active ingredient: 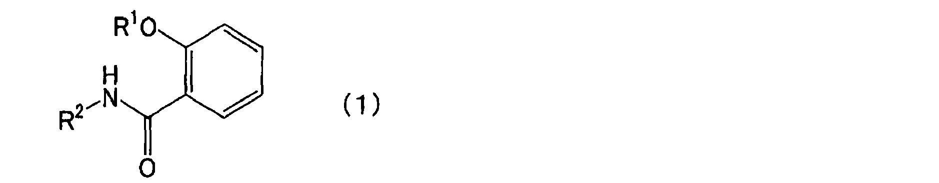 wherein R¹ represents a hydrogen atom or a C2 to C4 alkanoyl group and R² represents a group represented by the following formula (A), (B), (C), (D), (E), (F), or (G): 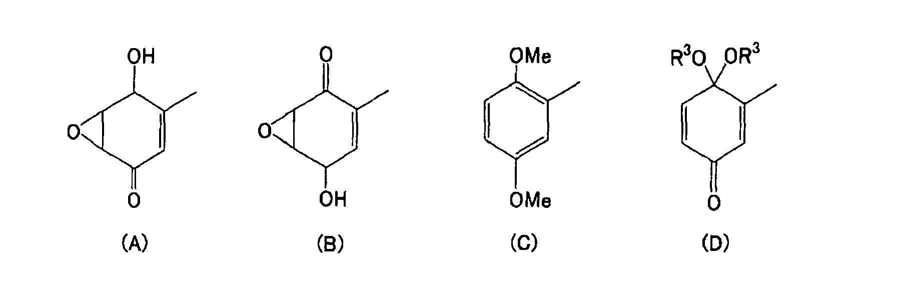 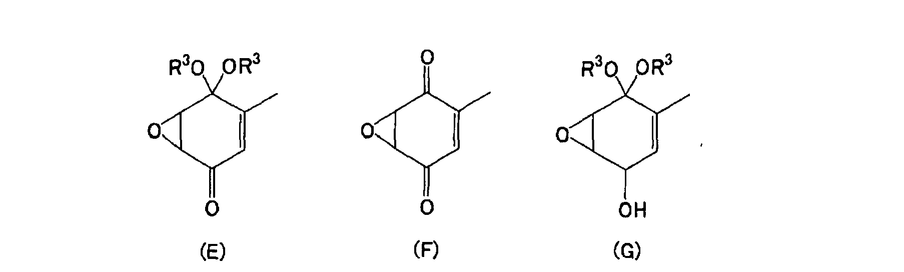 wherein R³ represents a C1 to C4 alkyl group.

20. The therapeutic agent of claim 19, wherein the compound is the following formula (1a) or formula (1b):

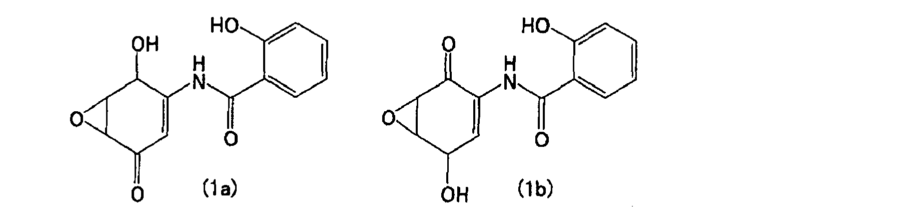

21. The therapeutic agent of claim 19, wherein the compound is the following formula (2):
